# Europäisches Patentamt

## European Patent Office

## Office européen des brevets

(19)

(11) Publication number: **0 127 649**
**B1**

(12)

# EUROPEAN PATENT SPECIFICATION

(45) Date of publication of patent specification: 02.09.87

(51) Int. Cl.⁴: **G 01 N 23/223,** G 01 N 33/34

(21) Application number: **83903792.6**

(22) Date of filing: **30.11.83**

(86) International application number:
**PCT/FI83/00075**

(87) International publication number:
**WO 84/02190 07.06.84 Gazette 84/14**

(54) PROCEDURE AND MEANS FOR MEASURING WITH THE AID OF A RADIOSOTOPE SOURCE THE DISTRIBUTION OF FILLERS IN A WEB.

(30) Priority: **01.12.82 FI 824141**

(43) Date of publication of application:
**12.12.84 Bulletin 84/50**

(45) Publication of the grant of the patent:
**02.09.87 Bulletin 87/36**

(84) Designated Contracting States:
**AT BE CH DE FR GB LI LU NL SE**

(56) References cited:
**DE-A-2 946 567**
**DE-A-3 219 962**
**DE-B-1 773 085**
**US-A-4 081 676**
**US-A-4 147 931**

**Paperi Puu 52, nr 4a, p. 145-51, 154-8 (April 1970) J. Kuusi "Determination of content and distribution of filler and coating materials in paper using radioisotope X-ray spectrometry".**

(73) Proprietor: **Robotest Oy**
**Teknologiantie 1**
**SF-79480 Kangaslampi (FI)**

(72) Inventor: **KUUSI, Juhani**
**Pihlajatie 12-14 C 20**
**SF-00270 Helsinki 27 (FI)**

(74) Representative: **Berglund, Gustav Arthur et al**
**AWAPATENT AB Box 5117**
**S-200 71 Malmö (SE)**

(56) References cited:
**Abstract in English from Paperchen, accession Number 50-06512 Tsellyuloza Burmaga Karton nr 3, 8 (1979) (Russ) Lienyanskii et al "Monitoring apparatus for determining filler content in a moving paper web.**
**Abstract in English from Paperchen, accession Number 46-03646 Sb. Tr. Ukp. N11 Isellyul - Bumazh, prom nr 17: 1974 p 123-126 (Russ) Gladaravskii et al "Use of X-rays for determining the filler content in paper".**
**Paper and Timber, No. 4A, 1970, pages 145 to 158**

Courier Press, Leamington Spa, England.

**0 127 649**

**Description**

The present invention concerns a method for measuring the distribution in the thickness direction of filler and/or coating materials of paper, cardboard or equivalent and the contents of said materials without destroying the sample, in said method the radiation emitted by an X-ray tube being used to excite in the material component under examination of the object under measurement its characteristic X-ray radiation, the intensity of this radiation being observed, and in which method measurements are carried out on both sides of the specimen under examination and in addition the contents of filler components are determined by X-ray radiation absorption measurements in order to eliminate the effects of said components interfering with the distribution measurement, and the base weight of the paper is determined by beta radiation absorption measurement or by another equivalent procedure.

Furthermore, the present invention concerns novel uses of the method.

When paper and paper machines are discussed in the following, reference is generally made both to paper and cardboard, and respectively both to paper and cardboard machines.

Fillers, which as a rule are mineral substances, are incorporated in the paper primarily for their effect of improving the printing technological properties. Fillers are most commonly used for printing papers. The filler addition improves the opacity, lightness, printer's ink absorption and surface smoothness of the paper.

The fillers influence in a particularly advantageous manner the quality of paper to be glazed.

It is known in the art to add filler material in two ways, either by mass filling or by coating. In the former procedure, the filler material is added in the form of suspension to the pulp sludge before the arrival of the sludge on the paper machine, whereby the filler material is admixed to the entire fibre material in the finished paper. In the latter procedure, a suitable glue substance is admixed to the filler material in the aqueous phase, such as starch or casein, whereafter the surface of the paper is brushed with this mixture in a continuous process.

The filler materials in paper tend to be non-uniformly distributed in the thickness direction of the paper, causing one-sidedness of the paper. The one-sidedness of paper manufactured on Fourdrinier machines is due to the fact that the fillers are washed out together with the water that is drained, from the lower part of the pulp web into the drainage water, whereby they become enriched in the upper part of the web. As is known in the art, endeavours have been made to reduce the problems of one-sidedness, not only by additives improving the retention, but also by gentle dewatering at the initial draining phase, which requires a longer dewatering time and therefore implies lengthening the wire section or reducing the speed of the paper machine.

In machines with a planar wire, the difficulties with the fines and filler distribution manifest themselves when papers for offset printing are manufactured. A high filler and fines content on the top surface of the paper causes dusting, which is a serious detriment in the offset process. In contrast, papers manufactured on a twin wire machine are considered well appropriate for offset printing. This is due to the symmetrical shape of the fines distribution and to equal leaching of both surfaces of the web owing to two-sided dewatering. It is in fact generally held that owing to more uniform fines distribution, the printing by offset on paper manufactured on a twin wire machine is more successful than that on paper manufactured on a Fourdrinier machine. Offset printability has indeed increased in significance because offset printing is increasingly replacing the letterpress printing procedure.

On the other hand, the filler content of the surfaces of the paper web cannot always be brought on desired level with a twin wire former, and even when using planar wires only the top side of the web (the side facing away from the wire) is satisfactory as to its filler content. The low filler content of the web surface is particularly problematic in so-called SC gravure papers. Attempts may be made to increase the filler content of the paper surfaces by increasing the filler content of the pulp in the headbox, but even with this expedient no satisfactory condition is achieved because of the above-mentioned poor retention characteristic of the filler and of its enriching in the inner parts of the paper. In addition, when the filler content in the headbox has to be increased, the consistency in the headbox is likely to become excessive so that it impairs the formation of the paper.

Modern high-speed printing presses impose particularly high requirements on the printing paper. These requirements are based on trouble-free operation of fast printing presses and on the appearance of the printing. The imprint is considerably influenced by the symmetry between the sides of the paper and the quality of the surfaces of the paper, which is naturally also influenced by the distribution of the fillers. Heretofore no procedures and means have been in use with which the filler distribution could have been measured even on line either on the paper machine, on the printing press or on the paper coating means. The object of the present invention is therefore to provide a new procedure and means suited, in addition to laboratory measurements, for the uses mentioned and which renders possible the control and adjustment of the manufacturing process on the paper machine on the basis of filler distribution measurements.

It is known in the art (e.g. the Finnish Patent No. 40587; inventors: Juhani Kuusi and Antti Lehtinen, applicant: Valmet Oy) to excite the characteristic X-ray radiation of the filler material by means of radiations (alfa, beta, gamma or X-ray radiation) penetrating into various depths in the paper, and in this way to gain information on the vertical distribution of the filler. The procedure has been described in greater detail in : J. Kuusi, Determination of Content and Distribution of Filler and Coating Materials in Paper Using

2

Radioisotope X-Ray Spectrometry, Paper and Timber No. 4A (1970) pages 145 to 158. All the examinations in the paper were based both on experimental X-ray fluorescence intensities measured from both sides of the paper sheet and on calculated intensity ratios, which were estimated from known paper filler or coating material distributions. The examinations concerned circumstances in which paper filler or coating material was composed of only one single filler or a mixture of constant composition. Variations in relation to each other of the filler component contents as is frequently the case in practice cause effects on the measured X-ray fluorescence intensities, the quantitative elimination of which is impossible by the procedures described in the paper. This has impeded the introduction in practice of the procedures.

As regards filler measurements, the state of art is illustrated in general by the publication of April 1982: Buchnea A., McNelles L. A., Hewitt J. S., The Application of X-Ray Absorption and Fluorescence Analysis to the Measurement of Paper Additives, Int. J. Appl. Radiat. Isot. Vol. 33, pp. 285 to 292 (1982), where a fluorescence and absorption technique is used to the purpose of determining the total contents of different fillers. The filler component total contents are determined by X-ray fluorescence measurements except for the one which has too low characteristic X-ray energy and which is measured by the X-ray absorption measurement. The measurements are based on the assumption that the fillers are uniformly distributed in the thickness direction of the paper. In practice, this is hardly ever the case. It is thus understood that in said publication, and likewise in the references cited therein and in its author's patent: "On-Line System for Monitoring Sheet Material Additives", U.S. Patent No. 4,081,676, March 1978, no endeavours whatsoever have been made to determine the important thickness-direction distribution, nor has it even been taken into account as a potential source of error in determination of the total filler content. It should be noted, however, that in the instances described in the paper the influence of said source of error is minimal.

The object of the present invention is to provide a method by which the thickness-direction filler distribution in the paper and the total filler contents can be determined either in the laboratory or directly on the paper machine (on line) also in the case that the contents of different filler components, e.g. $CaCO_3$, $TiO_2$, kaolin, talc or equivalent, are variable.

Procedures capable of determining the filler distribution and the total filler content directly on the paper machine are not in use at all. The object of the present invention is to provide an opportunity not only for immediate product quality control directly on the machine (on line), but also an entirely new possibility of controlling the paper manufacturing process, the significance of which is emphasized when endeavours are made to manufacture printing paper meeting ever greater quality requirements at lowest material costs. The object of the present invention is to achieve that the distribution can be measured and that it can also be controlled, which opens also a chance to develop the paper machine construction and the total control systems of paper machines.

In addition, it is an object of the invention to provide a procedure which is also suitable for quality control of the paper fed into fast modern printing presses, and possibly for the control and/or adjustment of the operation of said printing presses.

To achieve the aims presented in the foregoing and those which will become apparent later on, the method stipulated in claim 1 is used.

The method defined in the foregoing may be used, as claimed in claims 11, 13 and 14, e.g. on a paper machine, in on-line measurement for measuring the filler distribution in the thickness direction and the total filler content of paper. In addition, the results of measurement that are obtained can be used as feedback signals in the control system of the paper machine in the control of the filler distribution and/or of the total filler content of various filler materials. An advantageous application of the invention is in measurement, and possibly in the control, of the coating material content and/or coating material distribution in paper or cardboard that is either being coated in an on-line process or has been tested in a separate coating apparatus, in particular of its one-sidedness.

One potential application of the invention is the quality control of the paper being fed into a printing press and/or controlling, the operation of the printing press.

As has in part become apparent already in the foregoing, the inventive idea is that on both sides of the paper is measured the intensity of the characteristic X-ray radiation of the filler component excited with different radiation sources and possibly with different angles of incidence of the exciting radiation, this intensity furnishing information about the shape of the distribution. In addition in this X-ray fluorescence measurement it is possible to determine the intensity of the exciting radiation scattered back from the paper and which correlates, for instance, with the base weight of the paper. What is significant from the point of view of practical applications is that the contents of various filler components are measured by means of X-ray absorption measurements, making use of the primary radiation emitted by the radiation source and a radiation with desired absorption properties which has been derived from this source or from a source placed on the other side of the paper, with the aid of appropriate transformation targets. An auxiliary quantity is the absorption measurement signal of beta radiation used as routine in measurements on paper for determinations of base weight (in $g/m^2$) (fibres plus filler). Based on the results of the absorption measurements, it is possible by calculation to eliminate the effects of the variation of the different filler components' contents on the fluorescence measurements, and in this manner to determine the filler distribution and the contents of different filler components.

In the laboratory, the invention affords a chance for a rapid quality control of the paper, and thereby for the control of the manufacturing parameters with a given lead time. Particularly the filler distribution close to the surface layers of the paper has a remarkable significance for the printability of the paper. Moreover, a distribution of proper shape gives an opportunity to use filler in abundance, thereby lowering the total material costs. The procedures presently used in laboratories, such as dividing the paper into different layers by the aid of a tearing tape, incineration of layers and ash determination, are slower by one order of magnitude and more inaccurate than the procedure of the present invention.

Various embodiment examples of the invention and its physical and mathematical background are considered in the following in greater detail, reference being made to the figures of the drawing attached, to the details of which the invention is not confined.

Fig. 1 presents a typical filler distribution in paper manufactured on a Fourdrinier machine.

Fig. 2 presents mass absorption coefficients of some mineral filler and coating materials of paper and of water and of cellulose for low energy X-ray radiation.

Fig. 3 shows the main principle of the fluorescence measurement of the invention.

Figs. 4A and 4B present the principle of the fluorescence measurement of the invention with two different angles of incidence of the exciting radiation and angles of departure of the excited radiation.

Fig. 5A presents the distribution of filler components prior to coating the paper, while Fig. 5B presents the values of the same paper after the coating process.

Fig. 6A presents the principle of a fluorescence measuring apparatus.

Fig. 6B illustrates the absorption measurement with X-ray radiation.

Fig. 6C presents the principle of the absorption measuring means taking place with beta radiation.

Fig. 6D presents an arrangement alternative to Fig. 6C in the absorption measurement taking place with X-rays.

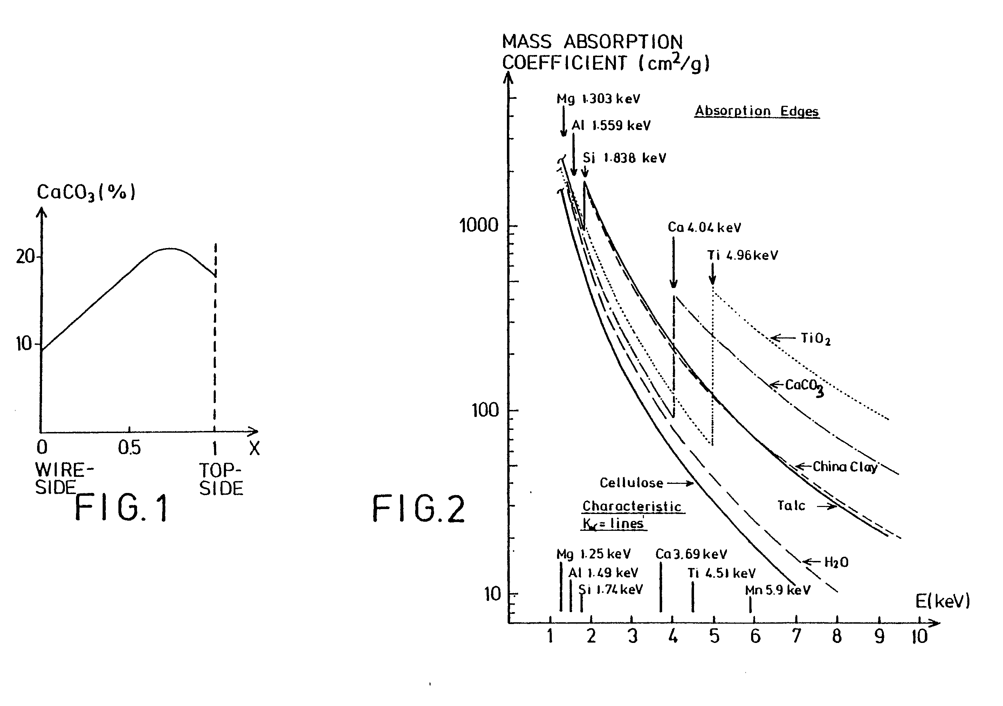

A typical filler distribution of paper in its thickness direction x can be seen in Fig. 1. The filler is least in quantity on the wire side. In this instance, the maximum is reached slightly above the centre-point of the paper (marked as 0.5 on the horizontal axis). The filler content decreases towards the top surface ($x=1$).

The attenuation (extinction) of X-ray, gamma and beta radiation in matter can generally be expressed by the exponential formula:

$$I = I_o \, e^{-\mu m},$$

where I is the intensity of the radiation that has gone through a mass course m ($g/m^2$), $I_o$ the original intensity of the radiation and $\mu$ ($cm^2/g$) is the absorption coefficient representing the extinction capacity of the material.

The absorption coefficients for low energy (1 to 10 keV) X-ray and gamma radiation (both are the same kind of electromagnetic radiation) of materials which are important in view of filler measurements are set forth in Fig. 2, plotted over energy. In the figure, the energy (in keV) has been plotted on the horizontal axis and the absorption coefficient $\mu$ (in $cm^2/g$) on the vertical axis. With the exception of a few discontinuous irregularities, the absorption coefficient and therefore also the extinction in the material decreases with increasing energy. However, some of the discontinuous jumps seen in the figure are of central importance in the embodiments of the invention. If we scrutinize the graph of the absorption coefficient of calcium carbonate ($CaCO_3$), we find that it descends smoothly throughout the range from 1 to 4 keV, until at 4.04 keV energy its value discontinuously increases to be tenfold and thereafter once more decreases smoothly with increasing energy of the radiation. The physical cause underlying this jump is as follows: in the range under consideration, X-ray and gamma radiation are attenuated in the material in the way that the energy of the radiation quanta transfers totally to electrons in the atoms, such electrons by virtue of the energy imparted to them being flung out from the atom, leaving behind a vacancy in the electron shroud. The energy of the X-ray or gamma quantum has to be higher than the binding energy holding the respective electron to its atom. When the energy of the radiation is lower than the 4.04 keV corresponding to the jump in the graph for $CaCO_3$, the radiation is not able to detach from the calcium atom the electrons of its inner shell (the K shell), which are the electrons most strongly bound to the atom. When the energy of the incident radiation surpasses this limit, its quanta can become absorbed in the substance by detaching electrons from the inner shell, and this exactly gives rise to the discontinuous increment of the absorption coefficient. The higher the atomic number of a substance—in practise usually the heavier it is—the higher is the energy at which is found this K absorption limit, i.e. the absorption limit corresponding to the K shell.

Thus, it is shown in Fig. 2 that the K absorption limit, due to titanium, of titanium dioxide ($TiO_2$) is located at the energy of 4.96 keV. In talc and kaolin, the element with the highest atomic number is silicon (Si), and therefore the absorption coefficient decreases steadily after the absorption limit of silicon at 1.8 keV with increasing energy of the radiation.

It is thus understood that when on a substance, for instance on calcium, radiation is directed which has an energy higher than the K absorption limit of calcium, vacancies will form on the inner electron shells of the atoms. When these are filled by electrons falling from outer shells, the substance emits its characteristic K X-ray radiation, the energy of which because of recoil losses is slightly lower than the energy of the K absorption limit. The strongest line of the calcium K has energy 3.69 keV, which has also been indicated on the energy axis in Fig. 2.

Said characteristic X-ray radiation of each element produced through absorption is utilized in a way known in the art in X-ray fluorescence analyses for determining the chemical composition of the specimens

being analyzed. In the present invention, said absorption is utilized towards determining the filler content of the paper's different layers and thus towards determining the filler distribution. In order that the determination of said distribution could be made free-enough of error from the viewpoint of the practical applications, the total contents of the different filler components in the paper must be known. This is found out, in the present invention, by the aid of absorption measurements.

If in the absorption measurements the extinction caused by paper containing filler is measured with two radiation energies which are as close as possible to the absorption limit of a given component in the way that one energy is above and the other below the limit, the difference in the extinction caused by the paper will furnish information about the content of this filler component. If the paper contains kaolin, talc, calcium carbonate and titanium oxide as fillers, the difference in the extinction of the 5.9 keV line emitted by the $^{55}$Fe radiation source and of the K line of titanium (4.51 keV) will furnish information primarily about the titanium-dioxide content (Fig. 2), the difference in the extinction of the difference of 4.51 keV (Ti K) and 3.69 keV (Ca K) radiations will furnish information primarily about the $CaCO_3$ content, and the absolute extinction of the 3.69 keV radiation, primarily about the combined content of talc and kaolin, these latter components having absorption components which at the last-mentioned point are clearly higher than the absorption coefficients of any other components of the paper, as Fig. 2 reveals.

For determining the contents of various filler components of paper, it is furthermore necessary to know the base weight of the whole paper, that is, its mass per unit area (in $g/m^2$). This is found by measuring the extinction in the paper of beta radiation e.g. from an $^{85}$Kr source. This is because the different components of paper cause equal extinction of beta radiation (i.e., of electrons thrown out by nuclei). The use of beta radiation for determining the base weight of paper is known in the art of paper technology and is altogether routine of its nature.

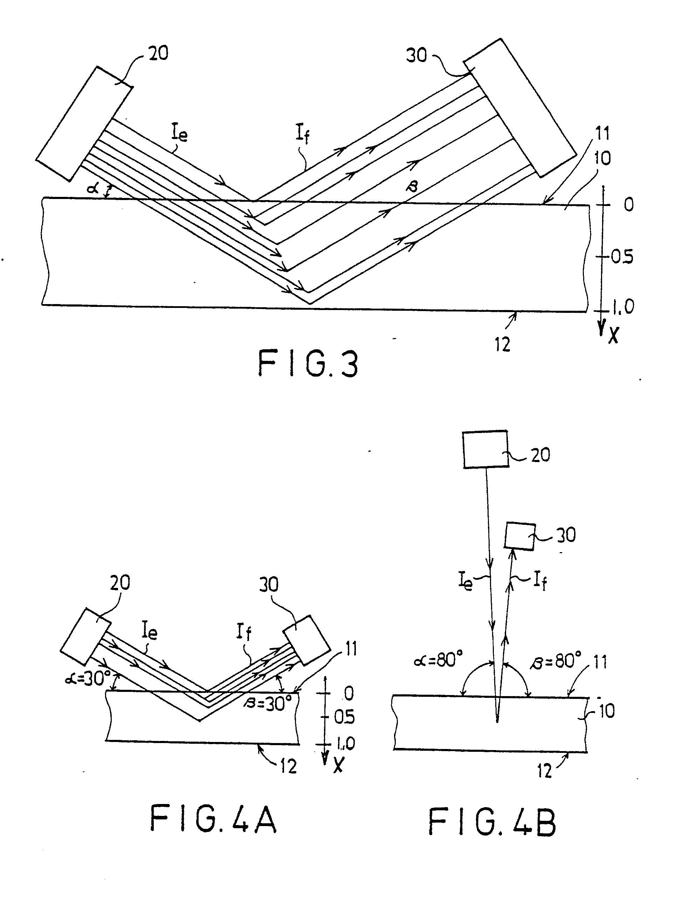

The fluorescence measurement used for the actual determination of the filler distribution is described more closely in the following referring to Fig. 3, in connection of which it shall be assumed that the base weight of the paper specimen 10 is 100 $g/m^2$ and that it contains, as uniformly distributed filler, 25% calcium carbonate. As shown in Fig. 3, the exciting radiation $I_e$—in the case under consideration, 5.9 keV radiation from the $^{55}$Fe source 20—impinges on the paper specimen 10 under the angle of incidence α and excites in the specimen 10 the characteristic radiation of calcium, of 3.69 keV. The detector 30 measuring this radiation $I_f$ observes the radiation departing under the angle β from the surface 11 of the specimen 10. Since the exciting radiation $I_e$ is attenuated as it proceeds in the paper specimen 10, it excites calcium radiation more efficiently in the adjacency of the top surface 11 which lies closer to the source 20 than in the adjacency of the lower, or back, surface 12. Since the excited characteristic radiation of calcium also suffers extinction in the specimen 10 to a given extent, the radiation excited adjacent to the top surface 11 has easier access to the detector 30.

Both circumstances just mentioned act in the direction that the greater part of the radiation detected by the detector 30, in the case of homogeneous filler distribution, comes from the top layers of the specimen 10, and therefore the topmost layers of the paper will be emphasized in the information thus obtained. The smaller the angles of incidence and departure α and β of the radiation, the greater are the differences in path length between the top surface and the lower surface 11 and 12, and the greater is the stress placed on the top surface 11 in the information gained by the detector 30. In this manner, it is possible by varying the angles of incidence and departure α and β to change the relative weight factors of different layers in the information that is measured. This is demonstrated by Figs. 4A and 4B and by the following Table 1.

TABLE 1

| | | 80° | 30° |
|---|---|---|---|
| Angle on incidence of the radiation (α) | | 80° | 30° |
| Angle of departure of the radiation (β) | | 80° | 30° |

| | Depth | Intensity | Intensity |
|---|---|---|---|
| Relative intensity of | 0.05 | 0.93 | 0.86 |
| information from different | 0.5 | 0.47 | 0.22 |
| depths in the paper | 0.95 | 0.23 | 0.06 |

In the above Table 1 is shown the relative intensity of the information received in fluorescence measurements (CaK line; $^{55}$Fe source) at various depths in the specimen when two different pairs of angles of incidence and of departure α, β of the radiation are used. The base weight of the paper is 100 $g/m^2$ and its $CaCO_3$ content 25%, assumed in this calculation example to be uniformly distributed in the vertical direction. On the depth scale, the surface has been denoted with the coordinate 0 and the back side of the paper with the value 1, making the coordinate of the centre 0.5.

The intensity values calculated in Table 1 reveal that the information is strongly weighted in favour of the top side, in other words, emphasizing that side, on which the measurement is performed. On changing the angles of incidence and departure of 80° in Fig. 4B one transfers to the angles α, β of 30° in Fig. 4A, this effect is even considerably strengthened. This is seen when, for instance, the values of the intensities obtained from the centre of the paper (0.5) are mutually compared (0.47 and 0.22).

Another way in which the relative weighting of the different layers may be varied is to use for excitation radiation sources with different energies. If in the case just considered the $^{55}$Fe source (5.9 keV) is replaced with a $^{238}$Pu source, of which the strongest radiation components have an energy 12—17 keV, the attenuation of this in the paper is so minimal that excitation will take place more or less uniformly throughout the thickness dimension of the specimen 10. For the excited radiation $I_f$, of course, the extinction effects are the same, independent of what radiation has affected the excitation.

If now the distribution of a given filler component in the thickness direction of the specimen 10 is not uniform but for instance as shown in Fig. 1, the intensities of the characteristic radiation of calcium measured on different sides of the paper are unequal and their difference reflects the one-sidedness of the distribution. A paper having a distribution substantially as in Fig. 1, with base weight 160 g/m$^2$ and calcium carbonate content about 20%, yielded with a $^{55}$Fe source and with angles α, β of incidence and departure, averaging 80°, for the ratio between different sides (top side/wire side): 470/410. On making the angles of incidence and departure smaller, this ratio increased as could be expected. An effect in the same direction was achieved using a $^3$H/Ti source, which emits softer (4.5 keV) radiation than the radiation (5.9 keV) of the $^{55}$Fe source.

In the following, the determination of the filler distribution on the basis of the results of measurement shall be considered.

The basic distribution as in Fig. 1 can be mathematically represented by a polynominal $y=ax^2+bx+c$, where y refers to filler content (vertical axis) and x to the coordinate in the vertical direction of the paper (horizontal axis). The coefficients a, b and c are found by fitting to a reference distribution. From a paper with reference distribution, the intensities of the characteristic radiation of calcium are determined from both sides of the paper to serve as reference values, and the X-ray absorption of the paper with a suitable source (e.g. $^{55}$Fe 5.9 keV), and the beta absorption (e.g. $^{85}$Kr source).

When now from an unknown specimen belonging to the same paper brand are measured the equivalent quantities, the differences between them and of the quantities measured from the reference paper will yield the filler distribution of the paper sample that is being measured, by mathematical methods, utilizing the known absorption coefficients of the different components. In the vicinity of the reference distribution, a measurement carried out with merely one pair of angles α, β or with one source already yields a rather reliable estimate on the distribution. The reliability and accuracy can be increased by varying the angles of incidence and of departure α, β or by using several sources 20 emitting energy $I_e$ with different energies. This naturally causes the mathematical processing to be more complicated.

In a case which was studied, for the reference polynominal representing the filler distribution was found $y=-42x^2+52.1x+6.7$, the unit of y and of the coefficients a, b and c being the CaCO$_3$ content (in %). It follows that the CaCO$_3$ according to the reference distribution, on the wire surface 12 (x=1) of the specimen 10, is 6.7% and on the top surface 11 (x=0), respectively, is 16.8%.

After the results of measurement for the intensity (I) of the characteristic radiation of calcium ($I_1$ is the value on the wire side 12, $I_2$ is the value on the top side 11) and the result of the X-ray absorption measurement (T) for the paper specimen under examination have been corrected by applying the reference graph, by the aid of the results of the beta absorption measurements to correspond to the base weight of the reference paper, the changes ($\Delta a$, $\Delta b$, $\Delta c$) of the coefficients of the distribution polynominal for the paper under examination can be calculated from the system of equations calculated from the reference polynominal:

$$\frac{\Delta I_1}{I_1}=0{,}6113 \cdot \Delta a+1{,}127 \cdot \Delta b+3{,}344 \cdot \Delta c$$

$$\frac{\Delta I_2}{I_2}=1{,}0403 \cdot \Delta a+1{,}832 \cdot \Delta b+2{,}781 \cdot \Delta c$$

$$\frac{\Delta T}{T} = \frac{1}{3} \cdot \Delta a+ \frac{1}{2} \cdot \Delta b+1 \cdot \Delta c$$

In the system of equations, $\Delta I$, $\Delta Ia$ and $\Delta T$ correspond to the values of the paper specimen 10 under examination and to those measured from the reference paper.

In tests that have been carried out, the new coefficients obtained from the system of equations were found to yield distributions in agreement with the distributions determined from the same paper specimens by activation analysis close to the reference distribution. It is obvious that a more accurate approximation is attained by a greater number of measurements, but the accuracy afforded by the procedure described in the foregoing is adequate in certain supervision applications.

If in the instance we are now considering, to the filler of the paper is added for instance kaolin in addition to calcium carbonate, as is frequently done intentionally or inadvertently (reused paper, etc.), the situation is significantly altered in the sense of measuring technology. This is because kaolin attenuates, in fluorescence measurements, both the exciting $I_e$ and the excited $I_f$ radiation (especially the last-mentioned), and as a result the variations of kaolin content affect to a certain degree the calcium carbonate measurements, even if the content and distribution of the latter should be constant in the specimen 10. The influence of kaolin on the results is however calculable and can be eliminated by the aid of the known absorption coefficients, provided that the kaolin content in the specimen 10 is known. This leads to the requirement of measuring technology that in connection with the measurements the contents of kaolin and other potential filler components have to be determined. This is possible by using in the absorption measurements radiation energies suitably selected, as has been described in the foregoing. It may be observed in this connection that of the commonly used fillers, talc and kaolin are materials of which the contents must be determined by the absorption technique. Fluorescence measurements do not succeed in normal conditions because in these substances the characteristic X-ray radiation even of the heaviest element, silicon (Si), is so weak that it is excessively attenuated in the specimen 10, in the air space and in the windows of standard detectors 30. Regarding $TiO_2$, which is occasionally used, the same methods can be applied as for $CaCO_3$, with the difference of course that the K line (4.51 keV) of titanium is excited and measured.

It is thus understood that in complicated cases the determination of the thickness-direction distribution of filler requires several X-ray fluorescence measurements on both sides of the specimen 10 and several absorption measurements. The intensity of the exciting radiation $I_e$ scattered back from the specimen 10, which correlates with several characteristics of the specimen paper may be used as a kind of control quantity in the measurements. In practical instances, when one is moving quite close to a given reference distribution, adequate accuracy is often achieved with rather few measurements.

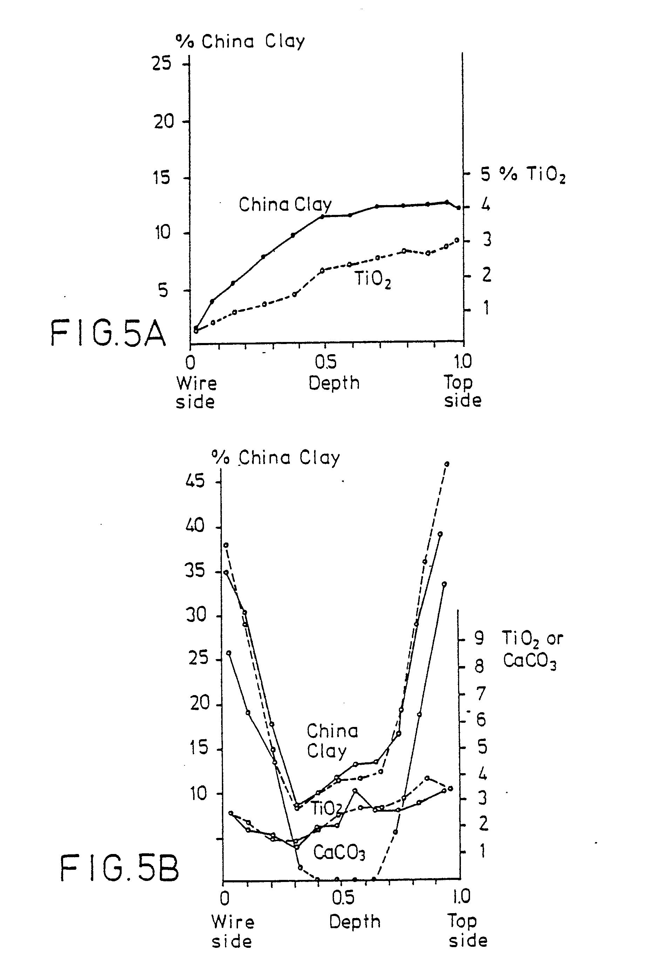

The printing characteristics of paper can be improved by coating the paper with the same substances that are used also as fillers. In that case the contents of mineral components in the surface layers of the paper increase greatly, as can be seen in the appended Figs. 5A and 5B. Since by the procedure of the invention information is gained about the distribution of the paper's mineral components in the paper and in particular about their content in the surface layers of the paper, it is also possible to determine the amount of coating in the coating layers and the difference in coating between the different sides of the paper by the procedure of the invention without destroying the specimen. If the paper is already coated, the filler distribution of the uncoated bottom paper naturally cannot be elicited any longer.

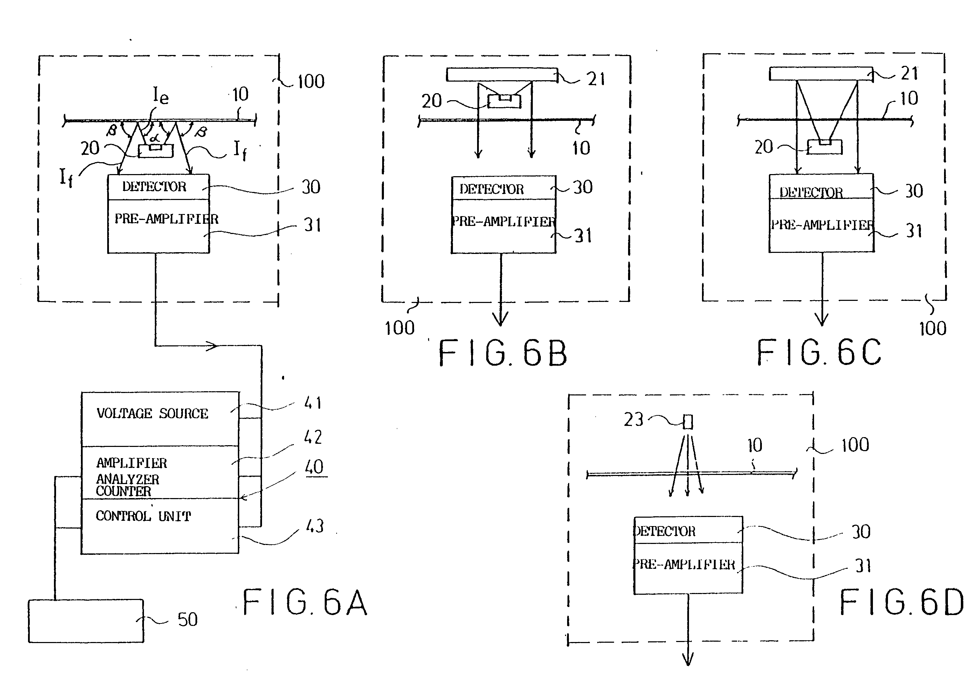

The part 100 isolated with interrupted lines in Figs. 6A, 6B, 6C and 6D is the measuring head, comprising radiation sources 20 and their transporting mechanisms known in themselves in the art, radiation transformation plates 21 and their transfer mechanisms (not presented in detail), a radiation detector 30 and a pre-amplifier 31. In the laboratory apparatus, the measuring head 100 is, for instance, an enclosed apparatus on the table, into which the paper specimen to be examined is conveyed by a suitable mechanism, this mechanism transporting the paper during one measuring cycle into one or several measuring positions.

In an on-line apparatus performing the measurements directly on the paper machine, the paper web passes through a measuring head 100 mounted on a measuring beam. The measuring head may be so constructed that it can be traversed across the paper web.

For detector 30 is primarily used a proportional counter. In certain instances, in particular in laboratory measurements, a semiconductor counter may also be used with a view to increasing the accuracy.

The measuring head 100 is connected to a measuring apparatus 40 comprising a voltage source 41, an amplifier and a counter, processor and display unit 42. A control unit 43 connected to the processor governs the performing of the measuring cycle and the processing of results.

In the laboratory version, the processor functions may be replaced with manual operations, and the results may also of course be processed manually or using an external computer 50. In fact, the measuring equipment external to the measuring head 100 is standard measuring equipment.

The extent of the equipment external to the measuring head 100 and of the computer programmes associated therewith is greatly dependent on the degree of automation and the standard of accuracy aimed at, and on the extent of the measuring range, that is on the number of different paper brands and the variation limits, within each brand, of the quantities which are measured.

Fig. 6A illustrates the exciting of the characteristic fluorescence radiation of a filler component ($CaCO_3$ or $TiO_2$) and its measuring on one side of the paper specimen 10. The radiation emitted by the radiation source 20 excites in the specimen 10 the characteristic X-ray radiation of a given element (Ca or Ti) of its filler part of which is going to the detector 30 and being counted. The detector 30 differentiates between the different kinds of radiation by their energy with such accuracy that from the measured pulse height distribution the contribution of each radiation component can be elicited by mathematical means. If it is desired to make the measurement with exciting radiations $I_e$ having different energies, the source of radiation 20 may be exchanged by the aid of a suitable mechanism. If, again, it is desired to employ different angles of incidence and departure $\alpha$, $\beta$ of the radiation with reference to the surface of the paper specimen 10, it is possible to move the source 20 laterally and possibly to use appropriate collineators or radiation beam detectors, known in themselves in the art.

Since for determining the filler distribution a fluorescence measurement has to be made on both sides of the specimen, in the laboratory version the paper specimen 10 must be turned over, or two measuring heads 100 carrying out measurement on different sides of the specimen 10 have to be used. When measurements are carried out directly on the paper machine, the latter alternative is the only possible.

Fig. 6B presents an arrangement by which absorption measurements are carried out with X-ray radiation of different energies. The radiation from the radio isotope source 20 is either scattered by an exchangeable target plate 21 or excites therein radiation appropriate for absorption measurements, this radiation passing partly through the paper specimen 10 into the detector 30. The 5.9 keV ($^{55}$Fe), 4.51 keV (Ti K) and 3.69 keV (Ca K) radiation components which were required above in our absorption measurement example are obtained with the aid of a $^{55}$Fe source by means of plastic (scattering), titanium or marble target plates 21. In certain instances the absorption measurements, too, may be performed with the aid of the source used in the fluorescence measurements, as shown in Fig. 6C.

As shown in Fig. 6C, the radiation that has passed from the source 20 through the specimen 10 is scattered from the backing plate 22 or excites therein radiation appropriate for absorption measurements, which partly passes through the specimen 10 into the detector 30. In this instance to the signal being measured is admixed the signal of the radiation excited in the paper specimen 10 by the source 20, this signal reducing the accuracy of measurement in certain cases.

Fig. 6D presents the absorption measurement, associated with the invention, used as routine in the papermaking industry towards base weight measurements and carried out with the aid of a beta radiation source 23, this measurement furnishing in distribution measurements according to the invention the auxiliary quantity which is indispensible in the processing of the results of measurement.

Detailed reference distributions which are indispensible for demonstrating and proving the practical applicability of the procedure of the present invention may be determined by neutron activation analysis of microtome sections made of paper. The technique is described in: Kuusi, J. and Lehtinen, A. J., "Neutron Activation Analysis of Microtome Cuts in Examination of Paper for its Filler Distribution", Pulp and Paper Magazine of Canada, 71, No. 3 (1970).

The procedure and the means described in the foregoing are suitable to be used either in laboratory measurements or in on-line measurements on a paper machine. In the last-mentioned use, the results obtained with the measuring apparatus may be used as feedback signals for guiding and/or controlling the papermaking process towards implementing a given desired filler distribution. A possible application of the invention is the use of the procedure in the measurement, and possibly even in the control, of the coating agent content and/or coating distribution either of paper or cardboard to be coated in an on-line process or of paper treated in separate coating means, in particular of its one-sidedness. Of the applications of the invention may further be mentioned the quality control of paper being fed into a printing press, and even the guiding or control of the operation of a printing press to the end of optimizing the printing quality and minimizing the trouble encountered in the operation of the printing press.

**Claims**

1. A method of non-destructive measuring of the distribution of filler and/or coating materials in the thickness direction of paper, cardboard or equivalent, and the content of said filler and/or coating materials, wherein radiation emitted by a radio-isotope source is used to excite the characteristic X-ray radiation of a material component to be examined, the intensity of said radiation being observed, wherein measurements are made on both sides of the paper under examination, wherein the contents of filler components and/or coating materials are determined by X-ray absorption measurements for eliminating the effects of components disturbing the observed X-ray fluorescence intensities, which are used for the distribution determinations, and wherein a beta radiation absorption measurement or equivalent procedure is carried out for obtaining the basis weight of the paper under examination, characterized in that said method comprises the steps of

making a number of absorption measurements, said number of measurements being at least equal to the number of different filler components and/or coating materials which have respectively different absorption coefficients, for determining the contents of the different filler components and/or the coating materials by X-ray radiation;

making a number of fluorescence measurements of the characteristic X-ray radiation of the material components excited in the paper by the radio-isotope source or sources; and

determining the distribution of fillers and/or coating materials by mathematical combination of the results from said absorption and fluorescence measurements.

2. The method as claimed in claim 1, characterized in that said method further comprises the steps of making X-ray fluorescence measurements and eliminating the effects exerted on the X-ray fluorescence measurements by variations of the different filler and/or coating materials' contents relative to each other by calculation with the aid of the total contents of the different filler and/or coating materials determined by X-ray absorption measurements in a manner whereby the thickness direction distributions of the different filler components in the paper are determinable from the X-ray fluorescence measurements.

3. The method as claimed in claim 1 or 2, characterized in that the distributions of filler or coating material components are measured by the use of radiation sources with different energy levels (E), the energy level (E) being selected such that it is slightly higher than the $K_a$ absorption edge energy of the material component to be examined.

4. The method as claimed in any one of claims 1—3, characterized in that said measurements are carried out at at least two different angles of incidence ($\alpha$) of said radiation.

5. The method as claimed in any one of claims 1—4, characterized in that said measurements are carried out at at least two different angles of departure ($\beta$) of the characteristic X-ray radiation excited in the paper by said radiation.

6. The method as claimed in any one of claims 1—5, characterized in that the angle of incidence ($\alpha$) of said radiation is equal in magnitude to the angle of departure ($\beta$) of the excited radiation relative to the plane of said paper on the same side of said paper.

7. The method as claimed in any one of claims 1—6, characterized in that said method further comprises the step of determining the intensity of the radiation from said radio-isotope source scattered back from the paper, which correlates with the basis weight of the paper, thereby providing an auxiliary quantity in the processing of results, in addition to X-ray fluorescence measurements.

8. The method as claimed in any one of claims 1—7, characterized in that the contents of various filler and/or coating materials are measured by X-ray absorption measurements utilizing the primary radiation emitted by said radiation source or radiation with certain absorption properties derived from said source or radiation from a source placed on the other side of said paper via a transformation target.

9. The method as claimed in any one of claims 1—8, characterized in that the filler and/or coating materials of said paper under examination are principally kaolin, talc and calcium carbonate or titanium dioxide or both of the latter, said method utilizing the absorption difference observed between the 5.9 keV radiation emitted by a $^{55}$Fe radiation source as the primary radiation source and that of the characteristic 4.51 keV K line excited in titanium primarily in determining the titanium dioxide content, utilizing the absorption difference observed between said K line of said titanium and the 3.69 keV K line of said calcium primarily in determining the $CaCO_3$ content, and using the information provided by the attenuation of said Ca-K line primarily for determining the combined content of talc and kaolin.

10. The method as claimed in any one of claims 1—9, characterized in that said method further comprises the step of measuring the attenuation in the paper under measurement of beta radiation emitted by an $^{85}$Kr source to determine the basis weight in $g/m^2$ of said paper.

11. Use of the method according to any one of claims 1—10 on a paper machine in on-line measurement for measuring the filler distribution in the thickness-direction and the total filler content of the paper.

12. The use according to claim 11, characterized in that the results of measurement obtained are used as feedback signals in the control system of a paper machine in controlling the filler distribution and/or the total filler content of different fillers.

13. Use of the method according to any one of claims 1—10 in measuring and possibly in controlling the coating material content and/or the distribution of the coating material, in particular its one-sidedness, in paper or cardboard to be coated in an on-line process or of paper processed in separate coating means.

14. Use of the method according to any one of claims 1—10 for quality control of the paper to be fed into a printing press and/or controlling the operation of the printing press.

## Patentansprüche

1. Verfahren zum zerstörungsfreien Messen der Verteilung von Füllmittel und/oder Beschichtungsmaterialen in der Dickenrichtung von Papier, Pappe oder dergleichen und das Gehalts des Füllmittels und/oder der Beschichtungsmaterialien, wobei von einer Radioisotopquelle ausgesandte Strahlung zur Anregung der charakteristischen Röntgenstrahlung eines zu untersuchenden Materialbestandnteils verwendet wird, wobei die Intensität der Strahlung beobachtet wird, wobei Messungen an beiden Seiten des Papiers bei der Untersuchung vorgenommen werden, wobei die Gehalte an Füllmaterialbestandteilen und/oder Beschichtungsmaterialien durch Röntgenabsorptionsmessungen bestimmt werden, um die Effekte von Komponenten zu eliminieren, welche die beobachteten Strahlenfluoreszenzintensitäten stören, welche für die Verteilungsbestimmungen verwendet werden, und wobei eine Betastrahlungsabsorptionsmessung oder ein äquivalentes Verfahren durchgeführt wird, um das Grundgewicht des untersuchten Papiers zu ermitteln, dadurch gekennzeichnet, dass das Verfahren folgende Schritte umfasst:

Durchführen einer Zahl von Absorptionsmessungen, wobei die Zahl der Messungen wenigstens gleich der Zahl an unterschiedlichen Füllmittelbestandteilen und/oder Beschichtungsmaterialien ist, welche jeweils unterschiedliche Absorptionskoeffizienten besitzen, um die Gehalte an unterschiedlichen Füllmittelbestandteilen und/oder der Beschichtungsmaterialien durch Röntgenstrahlung zu bestimmen;

Durchführung einer Zahl von Fluoreszenzmessungen der charakteristischen Röntgenstrahlung der Materialbestandteile, welche in dem Papier durch die Radioisotopenquelle bzw. -quellen angeregt wird; und

Bestimmung der Verteilung von Füllmitteln und/oder Beschichtungsmaterialien durch mathematische Kombination der Ergebnisse aus den Absorptions- und Fluoreszenzmessungen.

2. Verfahren nach Anspruch 1, weiterhin gekennzeichnet durch die Verfahrensschritte, dass

9

Röntgenfluoreszenzmessungen durchgeführt und die Effekte, welche auf die Röntgenfluoreszenzmessungen durch Variationen der Unterschiedlichen Füllmittel- und/oder Beschichtungsmaterialgehalte relativ zueinander ausgeübt werden, durch Berechnung unter Zuhilfenahme der Gesamtgehalte der unterschiedlichen Füllmittel und/oder Beschichtungsmaterialien eliminiert werden, welche mittels Röntgenabsorptionsmessungen in einer Weise bestimmt werden, bei der die Dickenrichtungsverteilung der unterschiedlichen Füllmittelbestandteile in dem Papier aus den Röntgenfluoreszenzmessungen bestimmbar sind.

3. Verfahren nach Anspruch 1 oder 2, dadurch gekennzeichnet, dass die Verteilungen von Füllmittel- und/oder Beschichtungsmaterialbestandteilen unter Verwendung von Strahlungsquellen mit unterschiedlichen Energieniveaus (E) gemessen werden, wobei das Energieniveau (E) derart ausgewählt wird, dass es geringfügig höher als die $K_\alpha$-Absorptionsrandenergie des zu untersuchenden Materialbestandteils ist.

4. Verfahren nach einem der Ansprüche 1—3, dadurch gekennzeichnet, dass die Messungen wenigstens mit zwei unterschiedlichen Einfallswinkeln (α) der Strahlung durchgeführt werden.

5. Verfahren nach einem der Ansprüche 1—4, dadurch gekennzeichnet, das die Messungen mit wenigstens zwei unterschiedlichen Ausfallswinkeln (β) der charakteristischen Röntgenstrahlung ausgeführt werden, welche in dem Papier durch die Strahlung angeregt wird.

6. Verfahren nach einem der Ansprüche 1—5, dadurch gekennzeichnet, das der Einfallswinkel (α) der Strahlung in seiner Grösse dem Ausfallswinkel (β) der erregten Strahlung relativ zu der Papierebene auf derselben Seite des Papiers gleich ist.

7. Verfahren nach einem der Ansprüche 1—6, dadurch gekennzeichnet, dass das Verfahren weiterhin den Verfahrensschritt enthält, dass die Intensität der von dem Papier zurückgestreuten Strahlung von der Radioisotopquelle bestimmt wird, welche mit dem Grundgewicht des Papiers korreliert, um dadurch eine Hilfsgrösse bei der Verarbeitung von Ergebnissen zusätzlich zu Röntgenfluoreszenzmessungen vorzusehen.

8. Verfahren nach einem der Ansprüche 1—7, dadurch gekennzeichnet, dass die Gehalte an verschiedenartigen Füllmitteln- und/oder Beschichtungsmaterialien durch Röntgenabsorptionsmessungen gemessen werden, wobei die Primärstrahlung, welche von der Strahlungsquelle ausgesandt wird, oder eine Strahlung mit bestimmten Absorptionseigenschaften, welche von der Quelle abgeleitet wird, oder eine Strahlung von einer auf der anderen Seite auf dem Papier angeordneten Quelle über ein Umformungstarget verwendet werden.

9. Verfahren nach einem der Ansprüche 1—8, dadurch gekennzeichnet, dass die Füllmittel und/oder Beschichtungsmaterialien des zu prüfenden Papiers im Prinzip Kaolin, Talk, Kalziumcarbonat oder Titandioxid oder die beiden letztegenannten sind, wobei das Verfahren primär zur Bestimmung des Titandioxidgehaltes die Absorptionsdifferenz verwendet, die zwischen der 5,9 keV Strahlung, welche von einer $^{55}$Fe-Strahlungsquelle als Primärstrahlungsquelle ausgesandt wird, und der der charakteristischen 4,51 keV K-Linie, welche in Titan angeregt wird, beobachtet wird, wobei primär zur Bestimmung des $CaCO_3$-Gehaltes die Absorptionsdifferenz verwendet wird, welche zwischen der K-Linie des Titans und der 3,69 keV K-Linie des Kalziums beobachtet wird, und wobei die von der Schwächung der Ca K-Linie gelieferte Information primär zur Bestimmung des zusammengesetzten Gehalts von Talk und Kaolin benutzt wird.

10. Verfahren nach einem der Ansprüche 1—9, dadurch gekennzeichnet, dass das Verfahren weiterhin den Verfahrensschritt aufweist, dass die Schwächung in dem Papier bei der Messung der Betastrahlung gemessen wird, welche von einer $^{85}$Kr-Quelle ausgesandt wird, um das Grundgewicht g/m² des Papiers zu bestimmen.

11. Verwendung des Verfahrens nach einem der Ansprüche 1—10 an einer Papiermaschine bei einer on-line Messung zur Messung der Füllmittelverteilung in der Dickenrichtung und des gesamten Füllmittelgehaltes der Papiers.

12. Verwendung nach Anspruch 11, dadurch gekennzeichnet, dass die erhaltenen Messergebnisse als Rückführsignale bei dem Regelungssystem einer Papiermaschine zur Steuerung der Füllmittelverteilung und/oder des gesamten Füllmittelgehalts unterschiedlicher Füllmittel verwendet werden.

13. Verwendung des Verfahrens nach einem der Ansprüche 1—10 bei der Messung und möglicherweise Steuerung des Beschichtungsmaterialgehalts und/oder der Verteilung des Beschichtungsmaterials, insbesondere dessen einseitiger Anordnung in zu beschichtendem Papier bei einer on-line Verarbeitung oder bei in separaten Beschichtungseinrichtungen verarbeitetem Papier.

14. Verwendung des Verfahrens nach einem der Ansprüche 1—10 zur Qualitätskontrolle des in einer Druckpresse einzuführenden Papiers und/oder zur Steuerung des Betriebs der Druckpresse.

**Revendications**

1. Procédé de mesure non destructive de la distribution de substances de remplissage et/ou de substances d'enduction dans le sens de l'épaisseur d'un papier, carton ou analogue, et de la teneur en de telles substances de remplissage et/ou de tolles substances d'enduction, procédé selon lequel on utilise le rayonnement émis par une source de radio-isotopes pour déclencher le rayonnement X caractéristique d'un composant de la substance devant étre examiné et l'on observe l'intensité dudit rayonnement, et

# 0 127 649

selon lequel on effectue des mesures sur les deux faces du papier examiné, on détermine les teneurs en composants de la substance de remplissage et/ou en substances d'enduction par des mesures d'absorption des rayons X afin d'éliminer les effect de composants perturbant les intensités de fluorescence par les rayons X observées, que l'on utilise pour les déterminations de distribution, et selon lequel on met en oeuvre une mesure d'absorption du rayonnement bêta ou une procédure équivalente, en vue d'obtenir le poids de base du papier examiné, caractérisé en ce que ledit procédé inclut les étapes consistant a:

réaliser un certain nombre de mesures d'absorption, ce nombre de mesures étant au moins égal au nombre des différents composants de la substance de remplissage et/ou des différentes substances d'enduction, qui possédent des coefficients d'absorption respectivement différents, en vue de la détermination des teneurs en ces différents composants de la substance de remplissage et/ou en ces substances d'enduction au moyen d'un rayonnement X;

réaliser un certain nombre de mesures de fluorescence du rayonnement X caractéristique des composants des substances et excité dans le papier par la ou les source(s) de radioisotopes; et

déterminer la distribution des substances de remplissage et/ou des substances d'enduction au moyen d'une combinaison mathématique des résultats fournis par lesdites mesures d'absorption et de fluorescence.

2. Procédé selon la revendication 1, caractérisé en ce que ledit procédé inclut en outre les étapes consistant â effectuer des mesures de fluorescence par les rayons X et à éliminer les effets produits sur ces mesures de fluorescence, par les variations relatives des teneurs en ces différentes substances de remplissage et/ou d'enduction, au moyen d'un calcul exécuté à l'aide des teneurs globales en ces différentes substances de remplissage et/ou d'enduction, déterminées par des mesures d'absorption du rayonnement X, d'une manière telle que les distributions des différents composants des substances de remplissage dans le sens de l'épaisseur du papier, pouvent être déterminées à partir des mesures de fluorescence par les rayons X.

3. Procédé selon la revendication 1 ou 2, caractérisé en ce que les distributions des composants de la substance de remplissage ou de la substances d'enduction sont mesurées par l'utilisation de sources de rayonnement possédant des niveaux d'énergie différentes (E), le niveau d'énergie (E) étant choisi de telle sorte qu'il soit légèrement supérieur à l'énergie de limite d'absorption $K_\alpha$ du composant du matériau devant être examiné.

4. Procédé selon l'une quelconque des revendications 1—3, caractérisé en ce que lesdites mesures sont exécutées sous au moins deux angles d'incidence différents ($\alpha$) dudit rayonnement.

5. Procédé selon l'une quelconque des revendications 1—4, caractérisé en ce que lesdites mesures sont exécutées sous au moins deux angles différents de départ ($\beta$) du rayonnement X caractéristique, déclenché dans le papier par ledit rayonnement.

6. Procédé selon l'une quelconque des revendications 1—5, caractérisé en ce que l'angle d'incidence ($\alpha$) dudit rayonnement possédé une valeur égale à l'angle de départ ($\beta$) du rayonnement déclenché, par rapport au plan dudit papier et sur la même face de ce dernier.

7. Procédé selon l'une quelconque des revendications 1—6, caractérisé en ce que ledit procédé inclut en outre l'étape consistant à déterminer l'intensité du rayonnement délivré par ladite source de radio-isotopes et diffusé parréflexion sur le papier, cette intensité étant en corrélation avec le poids de base du papier, ce qui fournit une grandeur auxiliaire lors de l'exploitation des résultats, en plus des mesures de fluorescence par les rayons X.

8. Procédé selon l'une quelconque des revendications 1—7, caractérisé en ce que les teneurs en différentes substances de remplissage et/ou d'enduction sont mesurées par des mesures d'absorption du rayonnement X, qui utilisent le rayonnement principal émis par ladite source de rayonnement ou un rayonnement présentant certaines propriétés d'absorption et dérivé de ladite source, ou un rayonnement délivré par une source placée de l'autre côté du papier, par l'intermédiaire d'une cible de transformation.

9. Procédé selon l'une quelconque des revendications 1—8, caractérisé en ce que les substances de remplissage et/ou d'enduction dudit papier examiné sont principalement du kaolin, du talc et du carbonate de calcium ou du bioxyde de titane ou ces deux derniers éléments, ledit procédé exploitant la différence d'absorption observée entre le rayonnement à 5,9 keV émis par une source de rayonnement $^{55}Fe$ en tant que source de rayonnement principal, et celui de la raie K caractéristique à 4,51 keV, excitée dans le titane, principalement pour la détermination de la teneur en bioxyde de titane, exploitant la différence d'absorption observée entre ladite raie K dudit titane et la raie K à 3,69 keV dudit calcium, principalement pour la détermination de la teneur en $CaCO_3$, et utilisant l'information fournie par l'atténuation de ladite raie Ca-K, principalement pour la détermination de la teneur combinée en talc et kaolin.

10. Procédé selon l'une quelconque des revendications 1—9, caractérisé en ce que ledit procédé inclut en outre l'étape consistant à mesurer l'atténuation dans le papier par la mesure du rayonnement beta émis par une source de $^{85}Kr$, pour déterminer le poids de base en $g/m^2$ dudit papier.

11. Utilisation du procédé selon l'une quelconque des revendications 1—10 dans une machine à papier pour une mesure en direct permettant de mesurer la distribution de la substance de remplissage, dans le sens de l'épaisseur, et la teneur totale en substance de remplissage du papier.

12. Utilisation selon la revendication 11, caractérisé en ce que les résultats de mesure obtenus sont utilisés en tant que signaux de réaction dans le système de commande d'une machine à papier pour le

11

**0 127 649**

réglage de la distribution de la substance de remplissage et/ou de la teneur totale en différentes substances de remplissage.

13. Utilisation du procédé selon l'une quelconque des revendications 1—10, pour la mesure et éventuellement le réglage de la teneur en substance d'enduction et/ou de la distribution de la substance d'enduction, en particulier son caractére unilatéral, dans du papier ou du carton devant étre enduit selon un processus en continu ou bien dans du papier traité avec des moyens séparés d'enduction.

14. Utilisation du procédé selon l'une quelconque des revendications 1—10, pour un contrôle de qualité du papier à introduire dans une presse d'imprimerie et/ou un réglage du fonctionnement de la presse d'imprimerie.

CaCO₃(%)

WIRE-SIDE
TOP-SIDE

FIG.1

MASS ABSORPTION
COEFFICIENT (cm²/g)

Mg 1.303 keV
Al 1.559 keV
Si 1.838 keV

Absorption Edges

Ca 4.04 keV
Ti 4.96 keV

1000

TiO₂

CaCO₃

100

China Clay

Cellulose

Talc

Characteristic
Kα - lines

H₂O

Mg 1.25 keV
Al 1.49 keV
Si 1.74 keV
Ca 3.69 keV
Ti 4.51 keV
Mn 5.9 keV

10

E(keV)

1  2  3  4  5  6  7  8  9  10

FIG.2

0 127 649

FIG. 3

FIG. 4A

FIG. 4B

FIG.5A

% China Clay

China Clay

TiO₂

5 % TiO₂

Wire side — Depth — Top side

FIG.5B

% China Clay

China Clay

TiO₂

CaCO₃

TiO₂ or CaCO₃

Wire side — Depth — Top side

3

FIG. 6A

FIG. 6B

FIG. 6C

FIG. 6D